(19) 

Europäisches
Patentamt
European
Patent Office
Office européen
des brevets

(11) **EP 4 257 554 A1**

(12) **EUROPEAN PATENT APPLICATION**
published in accordance with Art. 153(4) EPC

(43) Date of publication:
**11.10.2023 Bulletin 2023/41**

(21) Application number: **21915766.6**

(22) Date of filing: **28.12.2021**

(51) International Patent Classification (IPC):
*C01G 49/02* (2006.01)   *A61K 49/18* (2006.01)
*B82Y 5/00* (2011.01)   *B82Y 15/00* (2011.01)
*B82Y 40/00* (2011.01)

(52) Cooperative Patent Classification (CPC):
**A61K 49/18; B82Y 5/00; B82Y 15/00; B82Y 40/00;
C01G 49/02**

(86) International application number:
**PCT/KR2021/020042**

(87) International publication number:
**WO 2022/145968 (07.07.2022 Gazette 2022/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.12.2020  KR 20200185966**
**18.11.2021  KR 20210159441**

(71) Applicant: **ZTI Biosciences Co., Ltd.**
**Daejeon 34121 (KR)**

(72) Inventors:
• **CHANG, Hyung Seok**
  **Seoul 06284 (KR)**
• **PARK, Yong Sun**
  **Seoul 06743 (KR)**
• **RYU, Ji Young**
  **Yongin-si Gyeonggi-do 16840 (KR)**

(74) Representative: **Isarpatent**
**Patent- und Rechtsanwälte Barth**
**Charles Hassa Peckmann & Partner mbB**
**Friedrichstrasse 31**
**80801 München (DE)**

(54) **IRON OXIDE MAGNETIC PARTICLES**

(57)    The present invention provides iron oxide magnetic particles containing iron oxide and $MX_n$, wherein M as a transition metal containing electrons in a 6p orbital on the periodic table includes one or more selected from the group consisting of Tl, Pb, Bi, and Po, X includes one or more selected from the group consisting of F, Cl, Br, and I, and n is an integer of 1 to 6.

Fig. 1

**Description**

[Technical Field]

The present invention relates to iron oxide magnetic particles.

[Background Art]

Magnetic particles have been widely used in biomedical fields including cell labeling, magnetic resonance imaging (MRI), drug delivery, and thermotherapy. Superparamagnetic iron oxide magnetic particles among various types of magnetic particles have been widely studied in the field of biomedicine because of their high magnetic susceptibility and superparamagnetism.

In addition, since magnetic particles have a characteristic of generating heat when radiation or a magnetic field is applied thereto, they can also be used in contrast agents for magnetic resonance imaging (MRI), magnetic carriers for drug delivery in the field of nanomedicine, magnetic or radiation-based thermotherapy, and the like.

In the field of imaging diagnosis, iron oxide is a superparamagnetic contrast agent, and is proposed as a negative contrast agent. However, iron oxide particles have a strong hydrophobic attraction so that they may be well agglomerated with each other to form clusters or rapidly biodegrade when exposed to a biological environment. In addition, if the structure of iron oxide particles is not sufficiently stable, the original structure may be changed, and thus the magnetic properties thereof may be changed, and the iron oxide particles may have toxicity. Meanwhile, iodine is proposed as a positive contrast agent, but a formulation technology that increases the content per volume of the contrast medium has also been introduced due to a problem that liver/kidney toxicity occurs when it is used at high concentrations in order to enhance the contrast effect.

Meanwhile, in order to supplement the limitations of existing cancer treatment methods, thermotherapy based on radiation or electromagnetic fields has been proposed (Wust et al. Lancet Oncology, 2002, 3:487-497). One of the unique characteristics of cancer cells is that their ability to adapt to heat is significantly inferior to that of normal cells. Thermotherapy is an anticancer therapy that selectively annihilates cancer cells by raising the temperature of the cancer tissue and its surroundings to about 40 to 43°C by using the difference in thermal sensitivity between normal cells and cancer cells in this way. When injecting magnetic particles around cancer cells to apply a magnetic field from the outside, heat may be generated from the magnetic particles to annihilate the cancer cells in a short time. Since the magnetic field is not affected by skin tissue so that there is no limit to the penetration depth, heat may be selectively applied when magnetic particles are accumulated in cancer tissue in the body. Therefore, research on thermotherapy using magnetic particles has received a lot of attention.

Iron oxide magnetic particles are also mainly used as magnetic particles for thermotherapy. This is because iron oxide magnetic particles are materials having an indirect band gap in which energy equal to the amount of momentum used is converted into heat and released. Among them, $Fe_3O_4$ (magnetite) or a-Fe (ferrite)-based magnetic particles have biocompatibility, heat induction ability, chemical stability, and unique magnetic properties. Because of these characteristics, research as a self-heating element for thermotherapy of iron oxide magnetic particles is currently being actively conducted, and has been approved for medical use by the US FDA. However, $Fe_3O_4$ particles among iron oxide magnetic particles are nano-sized, and their crystalline phase easily changes to $\alpha$-$Fe_2O_3$, $\gamma$-$Fe_2O_3$, etc. depending on the conditions of the surrounding environment, and accordingly, the heating characteristics and their magnetic properties change, and thus there is a disadvantage of reducing the ability to generate heat. Research on Co, Ni, and Mg-based $MFe_2O_4$ (M=Co, Ni, and Mg) particles as another materials is being conducted, but this also has a disadvantage in that it is difficult to apply it to the inside of a living body due to a low exothermic temperature.

[Prior Art Document]

[Non-Patent Document]

Wust et al. Lancet Oncology, 2002, 3:487-497.

[Disclosure]

[Technical Problem]

A problem to be solved by the present invention is to provide iron oxide magnetic particles that can be used in various fields.

[Technical Solution]

In order to solve the above-described problem, the present invention provides iron oxide magnetic particles containing iron oxide and $MX_n$, wherein M as a transition metal containing electrons in a 6p orbital on the periodic table includes one or more selected from the group consisting of Tl, Pb, Bi, and Po, X includes one or more selected from the group consisting of F, Cl, Br, and I, and n is an integer of 1 to 6.

[Advantageous Effects]

The iron oxide magnetic particles of the present invention may have high reactivity to stimuli introduced from outside such as radiation, magnetic fields, and radio waves.

In addition, the contrast agent containing the iron oxide magnetic particles is used and can be applied to various diagnostic imaging devices, and can be administered in a small amount to obtain sufficient images.

The iron oxide magnetic particles of the present invention can have high structural stability due to a bond formed between iron oxide and a halogen compound and a transition metal element containing electrons in a 6p orbital.

[Description of Drawings]

FIG. 1 is transmission electron microscope (TEM) photographs of iron oxide magnetic particles of Example 3.
FIG. 2 is results of XPS component analysis of the iron oxide magnetic particles of Example 3.

[Modes of the Invention]

Hereinafter, various embodiments of the present invention will be described with reference to the accompanying drawings. It should be understood that the present invention is not limited to specific embodiments, and includes various modifications, equivalents, and/or alternatives of the embodiments of the present invention. In connection with the description of the drawings, similar reference numerals may be used for similar elements.

In this document, expressions such as "has", "may have", "includes", or "may include" refer to the presence of a corresponding feature (e.g., a numerical value, a function, an operation, or a component such as a part), and this does not preclude the existence of additional features.

In this document, expressions such as "A or B", "at least one of A or/and B", or "one or more of A or/and B" may include all possible combinations of the items listed together. For example, "A or B", "at least one of A and B", or "at least one of A or B" may refer to all cases of (1) including at least one A, (2) including at least one B, or (3) including both at least one A and at least one B.

The expression "configured (or set) to" used in this document may be used interchangeably with, for example, "suitable for", "having the capacity to", "designed to", "adapted to", "made to", or "capable of" depending on the circumstances. The term "configured (or set) to" does not necessarily mean "specifically designed to."

Terms used in this document are only used to describe a specific embodiment, and may not be intended to limit the scope of other embodiments. Singular expressions may include plural expressions unless the context clearly dictates otherwise. Terms used herein, including technical or scientific terms, may have the same meaning as commonly understood by a person of ordinary skill in the technical field described in this document. Among the terms used in this document, the terms defined in general dictionaries may be interpreted as having the meanings that are the same as or similar to those in the context of the related art, and unless explicitly defined in this document, they are not interpreted as having ideal or excessively formal meanings. In some cases, even terms defined in this document cannot be interpreted to exclude the embodiments of this document.

The embodiments disclosed in this document are presented for explanation and understanding of the disclosed technical content, and do not limit the scope of the present invention. Therefore, the scope of this document should be construed to include all changes or various other embodiments based on the technical idea of the present invention.

Hereinafter, preferred embodiments of the present invention will be described in detail. Prior to this, the terms or words used in this specification and claims should not be construed as being limited to the usual or dictionary meaning, and they should be interpreted as meanings and concepts consistent with the technical idea of the present invention based on the principle that the inventor can appropriately define the concepts of the terms in order to explain his/her invention in the best way.

Therefore, since the configurations of the embodiments described in this specification are only some of the most preferred embodiments of the present invention and do not represent all of the technical idea of the present invention, it should be understood that there may be various equivalents and modifications that can replace them at the time of this application.

Throughout the specification, when a certain part is said to "include" a certain component, it means that it may further include other components without excluding other components unless specifically stated otherwise.

In one aspect of the present invention, the term "about" has been used with the intention of including a slight numerical adjustment that falls within the scope of a manufacturing process error included in a specific numerical value or the scope of the technical idea of the present invention. For example, the term "about" means a range of $\pm 10\%$, $\pm 5\%$ in one aspect, and $\pm 2\%$ in another aspect of the value to which it refers.

Hereinafter, the present invention will be described in detail.

Iron oxide magnetic particles according to one embodiment of the present invention are iron oxide magnetic particles containing iron oxide and $MX_n$, wherein M as a transition metal containing electrons in a 6p orbital on the periodic table includes one or more selected from the group consisting of Tl, Pb, Bi, and Po, X includes one or more selected from the group consisting of F, Cl, Br, and I, and n is an integer of 1 to 6.

The term "iron oxide" is an oxide of iron, and for example, iron oxide may include one or more selected from the group consisting of $Fe_{13}O_{19}$, $Fe_3O_4$ (magnetite), $\gamma$-$Fe_2O_3$ (maghemite), $\alpha$-$Fe_2O_3$ (hematite), $\beta$-$Fe_2O_3$ (beta phase), $\varepsilon$-$Fe_2O_3$ (epsilon phase), FeO (wstite), $FeO_2$ (iron dioxide), $Fe_4O_5$, $Fe_5O_6$, $Fe_5O_7$, $Fe_{25}O_{32}$, ferrite type, and delafossite, but is not limited thereto.

The iron oxide magnetic particles specifically have magnetism as particles in which $MX_n$ is contained in iron oxide particles, and can amplify the contrast effect of iron oxide under conditions of magnetic fields or various radiation rays having a relatively low alternating magnetic field intensity and/or low frequency.

In addition, X may include a radioactive isotope of X or mixtures of radioactive isotopes of X. Specifically, it refers to a compound in which one or more atoms are replaced by an atom having the same atomic number, but an atomic mass or mass number different from the atomic mass or mass number commonly found in nature. Examples of isotopes suitable for inclusion in the compounds of the present invention include isotopes of fluorine, such as $^{18}$F; isotopes of chlorine, such as $^{36}$Cl; isotopes of bromine such as $^{75}$Br, $^{76}$Br, $^{77}$Br, and $^{82}$Br; and isotopes of iodine, such as $^{123}$I, $^{124}$I, $^{125}$I and $^{131}$I alone or in combination.

In addition, $MX_n$ may include one or more selected from the group consisting of $BiF_3$, $BiCl_3$, $BiBr_3$, and $BiI_3$.

According to the present invention, the fact that $MX_n$ is included in the iron oxide particles may mean that a physical or chemical bond is formed between the iron oxide particles and $MX_n$. Specifically, it may mean that $MX_n$ is disposed between iron oxide particles, or iron oxide and $MX_n$ are bonded through a hydrogen bond, $MX_n$ is formed on the surface of the iron oxide core by introducing a general coating method or introducing a doping method such as a diffusion process or an ion implantation process, or iron oxide crystal nuclei are formed inside $MX_n$ so that a shell structure is formed.

The iron oxide magnetic particles may have an average particle diameter (d50) of 0.1 nm to 1 $\mu$m. When the iron oxide magnetic particles have an average particle diameter (d50) of 0.1 nm to 100 nm, they may be used as a component of a contrast agent, anticancer agent, thermotherapy agent, or radiation therapy agent, and when the iron oxide magnetic particles have a size exceeding 100 nm, they may be applied to various in vitro medical fields.

M used in the present invention is a post-transition metal containing electrons in a 6p orbital on the periodic table and refers to four elements including Tl, Pb, Bi, and Po. In particular, Bi has the strongest diamagnetism among metals. Among the metals, it has the lowest thermal conductivity after mercury, and the lowest electrical conductivity of all metals. Bi, which does not contain lead, has high stability so that the compound is used in cosmetics and medical treatments.

When this is applied to the iron oxide magnetic particles of the present invention, it bonds to iron oxide in a trivalent form and is not decomposed from the particles in a magnetic field or strong frequency so that even when applied to the human body, stability of not being decomposed may be secured. In addition, according to the present invention, magnetic properties may be further enhanced by including Cu in iron oxides and including metal cations such as transition metals in which electrons are contained in the 6p orbital on the periodic table.

According to another embodiment of the present invention, the iron oxide magnetic particles may further contain a heavy atom-halogen compound. The term "heavy atom" includes, for example, atoms heavier than boron (B), such as Mn, Co, Cu, Se, Sr, Mo, Ru, Rh, Pd, Ag, Cd, Sn, Ba, Tl, and Pb, but is not limited thereto. Specifically, it may further include one or more selected from the group consisting of CuF, CuCl, CuBr, and CuI.

Presumably, when the iron oxide contains a post-transition metal-halogen compound containing electrons in the 6p orbital according to the present invention, the post-transition metal-halogen compound containing electrons in the 6p orbital may be disposed on the iron oxide as primary particles and have gaps (vacancies or pores). In addition, when the heavy atom-halogen compound, which is relatively smaller in size than the primary particles, is disposed on the iron oxide as secondary particles, it fills the gaps between the primary particles composed of the post-transition metal-halogen compound containing electrons in the 6p orbital, and thus it may buffer and absorb the distortion caused by the expansion and contraction of the particles. Therefore, destruction of the iron oxide magnetic particles or crystal destruction is less likely to occur, and as a result, it is difficult for the halogen compound to be separated from the particles, and as a result, it is considered that the stability of the iron oxide magnetic particles can be improved.

A transition metal-halogen compound containing electrons in a 6p orbital and a heavy atom-halogen compound which are contained in the iron oxide magnetic particle may have a weight ratio range of 1:0.2 to 1:1.5. Since the gaps between the particles may be filled most efficiently when they are contained within the above-described range, the effect capable of buffering and absorbing distortion due to expansion and contraction can be excellently secured.

In one embodiment, at least a portion of the surface of the iron oxide magnetic particles may be additionally coated with a hydrophilic polymer to form a complex. The hydrophilic polymer may be introduced to increase solubility in water and stabilization of the iron oxide magnetic particles according to one embodiment, or to enhance targeting or penetration force into specific cells such as cancer cells. Such a hydrophilic polymer may preferably have biocompatibility, and may include, for example, one or more selected from the group consisting of polyethylene glycol, polyethylene amine, polyethyleneimine, polyacrylic acid, polymaleic anhydride, polyvinyl alcohol, polyvinylpyrrolidone, polyvinyl amine, polyacrylamide, polyethylene glycol, phosphoric acid-polyethylene glycol, polybutylene terephthalate, polylactic acid, polytrimethylene carbonate, polydioxanone, polypropylene oxide, polyhydroxyethyl methacrylate, starch, dextran derivatives, amino sulfonic acids, sulfonic acid peptides, silica, and polypeptides, but is not limited thereto. If necessary, in the case of targeting cancer cells, peptides or proteins containing folic acid, transferrin, or RGD may be used as the hydrophilic polymer, and hyaluronidase or collagenase may be used to enhance penetration force to cells, but are not limited thereto.

In one embodiment, the iron oxide may be derived from complexes of iron and one or more compounds selected from the group consisting of an aliphatic hydrocarbon acid salt having 4 to 25 carbon atoms and an amine-based compound. Examples of the aliphatic hydrocarbon acid salt having 4 to 25 carbon atoms may include one or more selected from the group consisting of butyrate, valerate, caproate, enanthate, caprylate, pelargonate, caprate, laurate, myristate, pentadecylate, acetate, palmitate, palmitoleate, margarate, stearate, oleate, vaccenate, linoleate, (9,12,15)-linolenate, (6,9,12)-linolenate, eleostearate, tuberculostearate, arachidate, arachidonate, behenate, lignocerate, nervonate, cerotate, montanate, melissate, and a peptide salt containing one or more amino acids. These compounds may be used alone or in the form of a mixed acid salt of two or more thereof.

The metal component of the aliphatic hydrocarbon acid salt having 4 to 25 carbon atoms may include one or more selected from the group consisting of calcium, sodium, potassium and magnesium.

Examples of the amine-based compound may include one or more selected from the group consisting of methylamine, ethylamine, propylamine, isopropylamine, butylamine, amylamine, hexylamine, octylamine, 2-ethylhexylamine, nonylamine, decylamine, laurylamine, pentadecylamine, cetylamine, stearylamine, cyclohexylamine, dimethylamine, diethylamine, dipropylamine, diisopropylamine, dibutylamine, diamylamine, dioctylamine, di(2-ethylhexyl)amine, didecylamine, dilaurylamine, dicetylamine, distearylamine, methylstearylamine, ethylstearylamine, butylstearylamine, triethylamine, triamylamine, trihexylamine, trioctylamine, triallylamine, oleylamine, laurylaniline, stearylaniline, triphenylamine, N,N-dimethylaniline, dimethylbenzylaniline, monoethanolamine, diethanolamine, triethanolamine, dimethylaminoethanol, diethylenetriamine, triethylenetetramine, tetraethylenepentamine, benzylamine, diethylaminopropylamine, xylylenediamine, ethylenediamine, hexamethylenediamine, dodecamethylenediamine, dimethylethylenediamine, triethylenediamine, guanidine, diphenylguanidine, N,N,N′,N′-tetramethyl-1,3-butanediamine, N,N,N′,N′-tetramethylethylenediamine, 2,4,6-tris(dimethylaminomethyl)phenol, morpholine, N-methylmorpholine, 2-ethyl-4-methylimidazole, and 1,8-diazabicyclo (5,4,0) undecene-7 (DBU).

The iron oxide magnetic particles of the present invention may be prepared by adjusting the mol% of $MX_n$ to about 1 to 13 mol% of a complex of iron and one or more compounds selected from the group consisting of aliphatic hydrocarbons and aliphatic amines.

In one embodiment, the iron oxide magnetic particles may contain $MX_n$ at a weight ratio of 1:0.005 to 0.10, preferably 1:0.01 to 0.08, based on iron oxide. The ratio may be measured using inductively coupled plasma mass spectroscopy, which is metal content analysis equipment. When $MX_n$ is contained within the above-described range in the iron oxide magnetic particles of the present invention, excellent specific loss power may be secured, and high temperature change may be secured under an external alternating magnetic field or when radiation rays are irradiated.

The iron oxide magnetic particles as described above can secure high specific loss power while having high reactivity to stimuli introduced from the outside, such as radiation, magnetic fields, and radio waves, so that they can be effectively used for thermotherapy described later.

Presumably, a compound such as $MX_n$ of the present invention not only may increase the intensity of magnetization by combining with iron oxide, which is a magnetic material, but also increase the size or total amount of electromagnetic field energy that the compound can absorb, thereby enabling the amount of thermal energy emitted from the final iron oxide-based magnetic particles to be increased. This may improve or increase high thermal energy emission (conversion) efficiency (ILP: Intrinsic loss power) compared to existing iron oxide-based magnetic particles, even in the electromagnetic field energy environment of the relatively low- to middle-frequency (50 Hz to 200 kHz) bands as well as the existing high-frequency (200 kHz or more) range.

In addition, the contrast agent containing the iron oxide magnetic particles may be used and applied to various diagnostic imaging devices, and may be administered in a small amount to obtain sufficient images.

Furthermore, since the iron oxide magnetic particles have high structural stability due to the bond formed between iron oxide and the $MX_n$ compound, there is no concern about side effects that can be caused by each component, and they can be safely applied to the human body due to low toxicity.

The iron oxide magnetic particles according to one embodiment of the present invention may be used for the use of radiation therapy or thermotherapy for annihilating cancer cells.

Since the iron oxide magnetic particles according to the present invention have magnetism, they can be usefully used in diagnostic methods using magnetic properties.

According to one embodiment of the present invention, the present invention provides a cancer diagnosis method comprising the steps of (a) administering a composition containing the iron oxide magnetic particles to a patient suspected of cancer, and (b) detecting whether or not the magnetic particles inputted into the patient exist by using a magnetic resonance device. When the magnetic particles according to the present invention are administered, the contrast between the lesion and the normal tissue is clearly enhanced in, for example, MRI T1- and T2-weighted images, so that a visualized contrast effect can be confirmed. When the iron oxide magnetic particles of the present invention are administered, since cancer diagnosis can be performed without additional administration of a contrast agent, cancer diagnosis and treatment may be simultaneously performed with the iron oxide magnetic particles of the present invention.

When a cancer cell targeting material or a penetration force enhancing material is bonded to the iron oxide magnetic particles of the present invention, thermal diagnosis and treatment may be performed more efficiently under an external alternating magnetic field or radiation irradiation.

The iron oxide magnetic particles used in the contrast agent composition may be contained in an amount of 0.1 to 15% by weight, 1 to 15% by weight, 1 to 10% by weight, 3 to 10% by weight, or 4 to 8% by weight based on the total contrast agent composition.

The iron oxide magnetic particles are contained within the above-described range, and thus the iron oxide magnetic particles may not be accumulated in the body but may be discharged to the outside of the body, thereby significantly reducing toxicity as a contrast agent.

In one embodiment, the contrast agent may exhibit a contrast effect in a magnetic field having a frequency of 1 kHz to 1 MHz or less or an intensity of 20 Oe (1.6 kA/m) to 200 Oe (16 kA/m) or less. The alternating magnetic field irradiated after the contrast agent is administered to the subject may have a frequency of 1 kHz to 1 MHz or a frequency of 30 kHz to 120 kHz. In general, an alternating magnetic field of 1 MHz or more should be applied in order to convert a spin state from a singlet to a triplet, but in the present invention, the triplet transition is possible even under an alternating magnetic field of tens to hundreds of kHz. Also, the alternating magnetic field may have a magnetic field intensity of 20 Oe (1.6 kA/m) to 200 Oe (16.0 kA/m), 80 Oe (6.4 kA/m) to 160 Oe (12.7 kA/m), or 140 Oe (11.1 kA/m). The contrast agent according to one embodiment is useful in that it can be used even in an alternating magnetic field having a low magnetic field intensity and/or frequency that is harmless to the human body unlike conventional high-energy methods.

The contrast agent of the present invention has a feature that is not limited to devices that can be applied for image diagnosis. Since the contrast agent of the present invention has both a negative contrast agent component and a positive contrast agent component, it has high contrast to exhibit an excellent contrast effect. The iron oxide contrast agent of the present invention shows higher radiation absorption HU (hounsfield unit) value and CT contrast effect than conventional iodine-based (Iohexol or Iopamidol) or gold nano-CT contrast agents. Existing iodine-based contrast agents have been reported to show a value of 3000 HU (4.6 HU based on 1 mg) based on 647 mg/ml, and gold nanoparticles have been reported to show a value of about 5 to 50 HU based on 1 mg. Meanwhile, the iron oxide magnetic particles of the present invention shows a value of about 50 to 100 HU based on 1 mg.

In addition to the CT contrast effect, the present invention can be applied to X-ray imaging, magnetic resonance imaging (MRI), US, optical imaging, single photon emission computed tomography (SPECT), positron emission tomography (PET), magnetic particle imaging (MPI), flat imaging, and rigid, flexible or capsule endoscopy. Since the iron oxide magnetic particles according to the present invention can be used without limitation in various devices, for example, a patient can receive examinations of several devices at once after administering one dose of a contrast agent, and it may be possible to reduce unnecessary time for injecting other contrast agents according to conventional devices. For example, when a CT scan and an MRI scan are to be performed in the near future, as the CT contrast agent is mixed with the MRI contrast agent in the body of the subject, the test result may be unclear, and as the subject receives a different contrast agent for each test, the probability of causing toxicity increases. However, since the contrast agent of the present invention can be applied to various devices in a complex manner, such inconvenience can be reduced.

Another aspect provides a composition for diagnosing cancer including a contrast agent according to one embodiment.

The cancer may be gastric cancer, lung cancer, melanoma, uterine cancer, breast cancer, ovarian cancer, liver cancer, biliary tract cancer, gallbladder cancer, bronchial cancer, nasopharynx cancer, laryngeal cancer, pancreatic cancer, bladder cancer, colon cancer, rectal cancer, colorectal cancer, cervical cancer, brain cancer, bone cancer, skin cancer, blood cancer, kidney cancer, prostate cancer, thyroid cancer, parathyroid cancer, or ureter cancer.

The composition for diagnosing cancer may be administered to a subject in an oral or parenteral manner, and may include a pharmaceutically acceptable carrier so that it is suitable for each administration. Suitable pharmaceutically acceptable carriers and preparations are described in detail in Remington's work (Pharmaceutical Sciences 19th ed., 1995).

When the composition for diagnosing cancer is administered orally, it may be administered in solid preparations such as tablets, capsules, pills, and granules, or in liquid preparations such as solutions and suspensions.

When the composition for diagnosing cancer is administered parenterally, it may be administered by intravenous injection, subcutaneous injection, intramuscular injection, intraperitoneal injection, intralesional injection, intratumoral injection, or the like.

When the composition for diagnosing cancer is administered orally or parenterally as a liquid, it may be prepared as an aqueous solution or suspension using a commonly known solvent such as isotonic sodium chloride solution, Hank's Balanced Salt Solution, or Ringer's solution.

In one embodiment, the composition for diagnosing cancer may be for simultaneously treating cancer.

As mentioned above, the contrast agent of the present invention can annihilate cancer cells by thermotherapy. The term "thermotherapy" means exposing body tissues to a temperature higher than normal body temperature to annihilate lesion cells including cancer cells or to make these cells have higher sensitivities to radiation therapy or anticancer drugs. Cancer thermotherapy includes a systemic thermotherapy that increases the effect of cancer treatment in combination with radiation therapy/drug therapy and a local thermotherapy that annihilates cancer cells by injecting magnetic particles into target solid cancer and applying an external alternating magnetic field.

As such, since thermotherapy is capable of selectively annihilating cancer cells, it has an advantage of lowering side effects. However, existing thermotherapy technologies based on magnetic particles have problems in that the heating value of the particles themselves is low due to an external alternating magnetic field and the continuity thereof is limited so that the limitations of thermotherapy have been pointed out. The following two methods have conventionally been used in order to solve such problems:

(a) a method of increasing the intensity or frequency of an external alternating magnetic field in order to increase the heating phenomenon of the particles, or
(b) a method of increasing the concentration of particles to be injected into a living body.

However, (a) the method of increasing the intensity or frequency of the external alternating magnetic field may cause red spots around the skin and slight burns, wounds, inflammation, necrosis, etc. in regions with a lot of fat, may damage not only cancer tissues but also normal tissue cells, or may cause a result of lowering immunity. In addition, since such a method cannot avoid side effects due to harmfulness to the human body, it is prohibited to use it for pregnant women, patients with severe inflammation, patients with cardiac pacemakers, and patients with severe pleural effusion and ascites. As an alternative thereof, (b) the method of increasing the concentration of particles injected into a living body may increase the probability of accumulation of the particles in the body, and sometimes cause toxicity problems due to the chemical composition of the particle surface.

However, the iron oxide magnetic particles according to the present invention result in efficient heat generation when used for thermotherapy using an external alternating magnetic field or radiation equipment due to the effect of amplifying the internal quantum efficiency of iron oxide due to the difference in permittivity or electron capacitance by halogen groups. Accordingly, it may be possible to drastically lower the concentration of particles put into the living body compared to conventional iron oxide-based particles, and thereby also significantly improve bioaccumulation amount and toxicity problems. In conclusion, the present invention can dramatically overcome the disadvantages of the conventional technology, which were limited in use due to low heating value, despite the advantages of biocompatibility, chemical stability, and magnetic properties of iron oxide magnetic particles.

Hereinafter, Examples and the like will be described in detail to aid understanding of the present invention. However, embodiments according to the present invention can be modified in various different forms, and the scope of the present invention should not be construed as being limited to Examples below. The embodiments of the present invention are provided to more completely explain the present invention to those skilled in the art.

Examples

Examples 1 to 15: Synthesis of Iron Oxide Magnetic Particles containing $MX_n$

(a) Synthesis of iron-oleic acid or iron-oleylamine complex

To be the ratios of Tables 1 and 2 below, $FeCl_3 \cdot 6H_2O$, sodium oleate (28 mmol) (Examples 1 to 5 and 10 to 14) or oleylamine (28 mmol) (Examples 6 to 9), 200 ml of hexane, 100 ml of ethanol, and 100 ml of deionized water were mixed and reacted at 110°C for 6 hours with vigorous stirring. After cooling the reaction solution at room temperature, the transparent lower layer was removed using a separatory funnel, 100 ml of water was mixed with the brown upper organic layer and shaken, and then the lower water layer was removed again. This was repeated 3 times. The remaining brown organic layer was moved to a beaker and heated at 110°C for 4 hours so that hexane was removed.

(b) Synthesis of iron oxide magnetic particles containing $MX_n$

4.5 g (5 mmol) of the iron-oleic acid complex prepared in (a) above and 1.7 g (6 mmol) of oleic acid were mixed (Examples 1 to 5 and 10 to 14), or 4.208 g (5 mmol) of the iron-oleylamine complex prepared in (a) above and 1.6 g (6 mmol) of oleylamine were mixed (Examples 6 to 9). In addition, the types and contents of $MX_n$ in Tables 1 and 2 below were 7 ml of 1-eicosene and 13 ml of dibenzyl ether, respectively. The mixed solution was put into a round bottom flask and gas and water were removed in a vacuum state at 90°C for about 30 minutes. Nitrogen was injected and the temperature was raised to 200°C. Thereafter, the temperature was raised to 310°C at a rate of 3.3 °C/min and then reacted for 60 minutes. After cooling the reaction solution, it was moved to a 50 ml conical tube, and 30 ml of ethanol and hexane were injected at a ratio of 1:1, and then centrifugation was performed to precipitate particles. After washing the precipitated particles with 10 ml of hexane and 5 ml of ethanol, the obtained precipitate was dispersed in toluene or hexane. Here, dibenzyl ether is decomposed into benzyl aldehyde and toluene at a temperature of 150°C or higher, and participates in crystal formation by helping the formation of hydrogen bonds between iron oxo (-Fe-O-Fe-) and a transition metal element in which electrons are contained in the 6p orbital on the periodic table-halogen compound ($MX_n$) by the radical generated from aldehyde. The size of the prepared particles was about 6 to 7 nm.

[Table 1]

| Remark | Iron complex (iron-oleic acid or iron oleylamine content g) | $MX_n$ type (content g) | Weight ratio of $MX_n$ based on iron complex (input amount during preparation) | Weight ratio of MXn based on iron oxide (analysis value after material formation: based on ICP) |
|---|---|---|---|---|
| Example 1 | 4.501g (5 mmol) | $BiI_3$ 0.023 g (0.039 mmol) | 0.005 | 0.0045 |
| Example 2 | 4.501g (5 mmol) | $BiI_3$ 0.135 g (0.229 mmol) | 0.030 | 0.028 |
| Example 3 | 4.501g (5 mmol) | $BiI_3$ 0.270 g (0.458 mmol) | 0.060 | 0.057 |
| Example 4 | 4.501g (5 mmol) | $BiI_3$ 0.360 g (0.61 mmol) | 0.080 | 0.076 |
| Example 5 | 4.501g (5 mmol) | $BiI_3$ 0.45g (0.763 mmol) | 0.100 | 0.091 |
| Example 6 | 4.208g (5 mmol) | $BiI_3$ 0.021 g (0.036 mmol) | 0.005 | 0.0048 |
| Example 7 | 4.208g (5 mmol) | $BiI_3$ 0.126 g (0.214 mmol) | 0.030 | 0.029 |
| Example 8 | 4.208g (5 mmol) | $BiI_3$ 0.337 g (0.571 mmol) | 0.080 | 0.078 |
| Example 9 | 4.208g (5 mmol) | $BiI_3$ 0.42 g (0.714 mmol) | 0.100 | 0.097 |

[Table 2]

| Remark | Iron complex (iron-oleic acid content g) | $MX_n$ type (content g) | Weight ratio of $MX_n$ based on iron complex (input amount during preparation) | Weight ratio of MXn based on iron oxide (analysis value after material formation: based on ICP) |
|---|---|---|---|---|
| Example 10 | 4.501g (5 mmol) | $BiF_3$ 0.270 g (1.015 mmol) | 0.060 | 0.058 |
| Example 11 | 4.501g (5 mmol) | $BiBr_3$ 0.270 g (0.602 mmol) | 0.060 | 0.056 |
| Example 12 | 4.501g (5 mmol) | $BiCl_3$ 0.270g (0.856 mmol) | 0.060 | 0.058 |
| Example 13 | 4.501g (5 mmol) | $BiI_3$ 0.270 g (0.458 mmol) <br> CuI 0.270 g (1.416 mmol) | $BiI_3$ : 0.060 <br><br> CuI: 0.060 | $GdI_3$ : 0.058 <br><br> CuI: 0.057 |
| Example 14 | 4.501g (5 mmol) | $BiI_3$ 0.135 g (0.229 mmol) <br> CuI 0.270 g (1.461 mmol) | $BiI_3$ : 0.030 <br><br> CuI: 0.060 | $GdI_3$ : 0.028 <br><br> CuI: 0.057 |
| Com. Ex. 1 | 4.501g (5 mmol) | CuI 0.270 g (1.416 mmol) | 0.060 | 0.057 |
| Com. Ex. 2 | 4.501g (5 mmol) | CuBr 0.270 g (1.882 mmol) | 0.060 | 0.058 |

(c) Preparation of iron oxide magnetic particles coated with hydrophilic ligand (polyacrylic acid)

While 2 g of polyacrylic acid and 40 ml of tetraethylene glycol were heated at 110°C, 150 mg of the iron oxide magnetic particles containing $MX_n$ of Examples and Comparative Examples above dispersed in 5 ml of hexane were injected with a syringe. It was stirred and reacted at 280°C for 8 hours. After cooling the reaction solution, 20 ml of 0.01 N HCl was put thereinto and particles attracted to the magnet were collected. After repeating this twice, a precipitate was obtained using ethanol and finally dispersed in water.

Experimental Example: XPS and TEM analysis

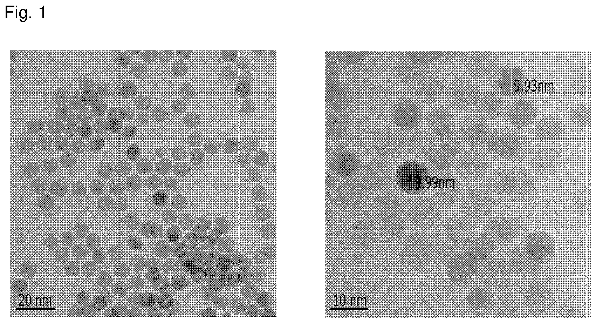

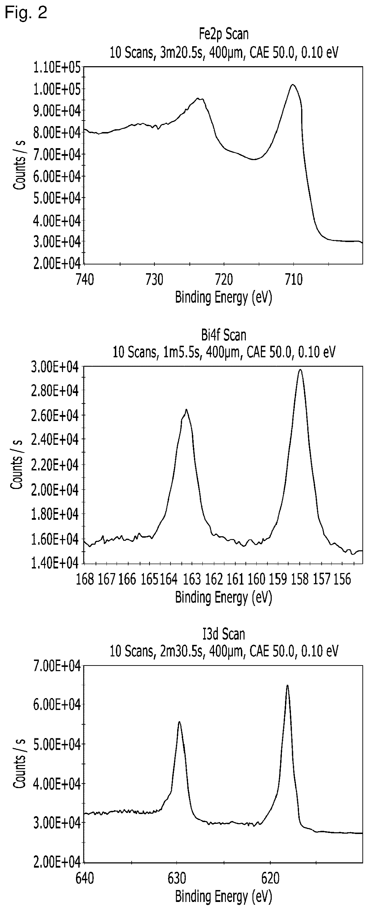

The TEM photographs of iron oxide magnetic particles of Example 3 above are shown in FIG. 1. In addition, the results of XPS component analysis of the iron oxide magnetic particles of Example 3 above are shown in FIG. 2.
Experimental Example: Analysis of temperature change according to the weight ratio of iron oxide and $MX_n$ under an external alternating magnetic field
After coating the particles prepared in Examples and Comparative Examples above with polyacrylic acid, which is a hydrophilic ligand, self-induction heating ability was tested. After diluting each of Examples and Comparative Examples in deionized water to a concentration of 20 mg/ml, an alternating magnetic field was applied, and the temperature changes were measured using a thermocouple (OSENSA, Canada) (alternating current frequency and magnetic field intensity used: f = 108.7 kHz, H = 11.4 kA/m). The results are shown in Table 3 below.

A system of performing heating by inducing an alternating magnetic field consists of four main subsystems of (a) a variable frequency and amplitude sine wave function generator (20 MHz Vp-p, TG2000, Aim TTi, USA), (b) a power amplifier (1,200 Watt DC Power Supply, QPX1200SP, Aim TTi, USA), (c) an induction coil (number of revolutions: 17, diameter: 50 mm, height: 180 mm) and a magnetic field generator (Magnetherm RC, nanoTherics, UK), and (d) a temperature change thermocouple (OSENSA, Canada).

[Table 3]

|  | Temperature reached per unit time (Standard: 1 minute) Start measurement at 25°C |
| --- | --- |
| Example 1 | 33 |
| Example 2 | 55 |
| Example 3 | 78 |
| Example 4 | 60 |
| Example 5 | 30 |
| Example 6 | 28 |
| Example 7 | 50 |
| Example 8 | 54 |
| Example 9 | 27 |
| Example 10 | 66 |
| Example 11 | 53 |
| Example 12 | 48 |
| Example 13 | 85 |
| Example 14 | 80 |
| Comparative Example 1 | 86 |
| Comparative Example 2 | 57 |

Experimental Example: Measurement of Specific Loss Power (SLP)

Since the heating value of particles varies depending on the physical and chemical properties and the intensity and frequency of an external alternating magnetic field, most research results show the heating ability of particles as SLP and ILP. SLP is the electromagnetic force lost per unit of mass, and expressed in Watts (W) per kg. Since the conditions of f (frequency) and H (magnetic field intensity) may be different for each experiment, the thermotherapy effects between the particles can be compared by converting the SLP value into an ILP value using the equation [ILP= SLP/(f H$^2$)].

For the SLP measurement, an alternating magnetic field generator (Magnetherm RC, Nanotherics) of a series resonant circuit controlled by a pick-up coil and an oscilloscope was used. The measurement was performed under adiabatic conditions of f = 108.7 kHz and H = 11.4 kA/m, and the temperatures were measured using an optical fiber IR probe.

SLP values were measured by adjusting the particles of Examples and Comparative Examples to a concentration of 20 mg/ml. The results are shown in Table 4 below.

[Table 4]

|  | ILP value |
| --- | --- |
| Example 1 | 1.30 |
| Example 2 | 5.28 |
| Example 3 | 8.98 |
| Example 4 | 6.08 |
| Example 5 | 1.17 |

(continued)

|  | ILP value |
|---|---|
| Example 6 | 1.09 |
| Example 7 | 4.73 |
| Example 8 | 5.18 |
| Example 9 | 1.05 |
| Example 10 | 6.93 |
| Example 11 | 5.08 |
| Example 12 | 4.22 |
| Example 13 | 9.47 |
| Example 14 | 9.50 |
| Comparative Example 1 | 9.81 |
| Comparative Example 2 | 5.14 |

Experimental Example: In Vivo Cancer Treatment Effect Checking Test

It was confirmed that cell annihilation by thermotherapy using the particles of Examples and Comparative Examples of the present invention effectively occurred even in vivo. After Hepg2 cells were transplanted into Balb/c nude mice, when the size of the cancer tissue was about 100 mm$^3$, 150 $\mu$l of an aqueous solution obtained by dispersing the compositions containing Examples and Comparative Examples in deionized water was subcutaneously administered, and then an alternating magnetic field generator (100 kHz, 80 G) was applied for 30 minutes to perform thermotherapy and check the volume of the cancer for 28 days. In the case of the control group below, the tumor sizes was measured when no separate treatment was performed.

The results are shown in Table 5 below.

[Table 5]

| Remark (Specific week) | Final tumor size after treatment (mm$^3$) | | | | |
|---|---|---|---|---|---|
|  | (Week 0) | (Week 1) | (Week 2) | (Week 3) | (Week 4) |
| Example 1 | 102 | 142 | 180 | 201 | 365 |
| Example 2 | 98 | 80 | 56 | 53 | 41 |
| Example 3 | 97 | 70 | 32 | 30 | 24 |
| Example 4 | 100 | 77 | 53 | 48 | 39 |
| Example 5 | 103 | 145 | 185 | 206 | 382 |
| Example 6 | 99 | 150 | 189 | 210 | 391 |
| Example 7 | 101 | 85 | 65 | 61 | 51 |
| Example 8 | 102 | 82 | 61 | 58 | 45 |
| Example 9 | 103 | 153 | 191 | 211 | 401 |
| Example 10 | 98 | 75 | 51 | 44 | 35 |
| Example 11 | 101 | 80 | 67 | 64 | 54 |
| Example 12 | 103 | 87 | 71 | 69 | 58 |
| Example 13 | 99 | 68 | 23 | 20 | 18 |
| Example 14 | 100 | 63 | 21 | 15 | 13 |
| Com. Ex. 1 | 98 | 65 | 21 | 18 | 15 |

(continued)

| Remark (Specific week) | Final tumor size after treatment (mm$^3$) | | | | |
|---|---|---|---|---|---|
| | (Week 0) | (Week 1) | (Week 2) | (Week 3) | (Week 4) |
| Com. Ex. 2 | 101 | 74 | 51 | 31 | 24 |
| Control group | 97 | 171 | 290 | 440 | 730 |

Experimental Example: Radiation Absorption Coefficient (HU) Measurement

Radiation (X-ray) absorption coefficients (HU) were measured using the particles of Examples, Comparative Examples and Control group above, and the results are shown in Table 6 below. At this time, Bruker's Skyscan 1172 Micro CT was used as a measuring device, and the radiation absorption coefficients (HU) were calculated as follows.

CT Hounsfield Unit (HU, X-ray Absorption Coefficient)

$$HU = 1000 \times \frac{\mu - \mu_{water}}{\mu_{water} - \mu_{air}}$$

($\mu$: Relative linear attenuation coefficient)

[Table 6]

| Remark | Radiation absorption coefficient (HU) per unit weight (based on 1 mg) |
|---|---|
| Example 1 | 57 |
| Example 2 | 79 |
| Example 3 | 96 |
| Example 4 | 87 |
| Example 5 | 50 |
| Example 6 | 48 |
| Example 7 | 70 |
| Example 8 | 75 |
| Example 9 | 43 |
| Example 10 | 91 |
| Example 11 | 73 |
| Example 12 | 63 |
| Example 13 | 110 |
| Example 14 | 105 |
| Comparative Example 1 | 97 |
| Comparative Example 2 | 18 |
| Control group 1 (iron oxide Fe$_3$O$_4$, see reference: Sci Rep., 8, 12706 (2018)) | About 10 |

Experimental Example: Thermogravimetric Analysis (TGA) of Iron Oxide Magnetic Particles

In order to examine the thermal stability of the iron oxide magnetic particles of the present invention (purpose is to confirm how stably the halogen elements are bound in the iron oxide magnetic particles), thermogravimetric analysis (TGA) was performed by using Scinco's S-1000. Specifically, the particles of Examples, Comparative Examples and Control group were subjected to TGA measurement up to 200°C at a rate of 20/min under nitrogen to perform comparison. The results are shown in Table 7.

[Table 7]

| Remark | Weight reduction rate (%) |
|---|---|
| Example 1 | 5.7 |
| Example 2 | 3.8 |
| Example 3 | 2.1 |
| Example 4 | 2.5 |
| Example 5 | 5.3 |
| Example 6 | 6.1 |
| Example 7 | 3.5 |
| Example 8 | 3.3 |
| Example 9 | 6.1 |
| Example 10 | 2.3 |
| Example 11 | 4.1 |
| Example 12 | 3.9 |
| Example 13 | 0.9 |
| Example 14 | 1.6 |
| Comparative Example 1 | 3.0 |
| Comparative Example 2 | 6.0 |
| Control group 1 (iron oxide $Fe_3O_4$) | 15 |
| Control group 2 (doped with 6% by weight of iron oxide-KI) | 17 |
| Control group 3 (doped with 6% by weight of iron oxide-$MgI_2$) | 13 |

Experimental Example: Microwave Stability Analysis of Iron Oxide Magnetic Particles

The particles of the Examples, Comparative Examples, and Control groups were irradiated for 15 minutes each at conditions of 2,400 to 2,500 MHz and 1000 W using a microwave device made in the US by CEM. After microwave irradiation, the contents of halogen elements were measured in a prodigy High Dispersion ICP measuring instrument equipped with a halogen option from A Teledyne Leeman Labs to confirm whether the particles were decayed or not. The results are shown in Table 8.

[Table 8]

| Remark | Measured halogen elements (ppm) |
|---|---|
| Example 1 | 18 |
| Example 2 | 8 |
| Example 3 | 4 |
| Example 4 | 7 |
| Example 5 | 19 |
| Example 6 | 20 |
| Example 7 | 9 |

(continued)

| Remark | Measured halogen elements (ppm) |
| --- | --- |
| Example 8 | 10 |
| Example 9 | 21 |
| Example 10 | 6 |
| Example 11 | 12 |
| Example 12 | 11 |
| Example 13 | 3 |
| Example 14 | 5 |
| Comparative Example 1 | 25 |
| Comparative Example 2 | 30 |
| Control group 1 (iron oxide $Fe_3O_4$) | 0 |
| Control group 2 (doped with 6% by weight of iron oxide-KI) | 65 |
| Control group 3 (doped with 6% by weight of iron oxide-$MgI_2$) | 57 |

## Claims

1. Iron oxide magnetic particles containing iron oxide and $MX_n$,
   wherein M as a transition metal containing electrons in a 6p orbital on the periodic table includes one or more selected from the group consisting of Tl, Pb, Bi, and Po, X includes one or more selected from the group consisting of F, Cl, Br, and I, and n is an integer of 1 to 6.

2. The iron oxide magnetic particles of claim 1, wherein X includes a radioactive isotope of X or mixtures of radioactive isotopes of X.

3. The iron oxide magnetic particles of claim 1, wherein at least a portion of the surface of the iron oxide magnetic particles is additionally coated with a hydrophilic polymer to form a complex.

4. The iron oxide magnetic particles of claim 1, wherein the iron oxide is derived from complexes of iron and one or more compounds selected from the group consisting of an aliphatic hydrocarbon acid salt having 4 to 25 carbon atoms and an amine-based compound.

5. The iron oxide magnetic particles of claim 1, wherein the iron oxide includes one or more selected from the group consisting of $Fe_{13}O_{19}$, $Fe_3O_4$ (magnetite), $\gamma$-$Fe_2O_3$ (maghemite), $\alpha$-$Fe_2O_3$ (hematite), $\beta$-$Fe_2O_3$ (beta phase), $\varepsilon$-$Fe_2O_3$ (epsilon phase), FeO (wstite), $FeO_2$ (iron dioxide), $Fe_4O_5$, $Fe_5O_6$, $Fe_5O_7$, $Fe_{25}O_{32}$, ferrite type, and delafossite.

6. The iron oxide magnetic particles of claim 3, wherein the hydrophilic polymer includes one or more selected from the group consisting of polyethylene glycol, polyethylene amine, polyethyleneimine, polyacrylic acid, polymaleic anhydride, polyvinyl alcohol, polyvinylpyrrolidone, polyvinyl amine, polyacrylamide, polyethylene glycol, phosphoric acid-polyethylene glycol, polybutylene terephthalate, polylactic acid, polytrimethylene carbonate, polydioxanone, polypropylene oxide, polyhydroxyethyl methacrylate, starch, dextran derivatives, amino sulfonic acids, sulfonic acid peptides, silica, and polypeptides.

7. The iron oxide magnetic particles of claim 1, wherein the iron oxide magnetic particles contain $MX_n$ at a weight ratio of 1:0.005 to 0.10 based on iron oxide.

8. The iron oxide magnetic particles of claim 1, wherein the particles have a diameter of 0.1 nm to 1,000 nm.

9. The iron oxide magnetic particles of claim 1, wherein the iron oxide magnetic particles are used at a frequency of 1 kHz to 1 MHz.

10. The iron oxide magnetic particles of claim 1, wherein the iron oxide magnetic particles are used in a magnetic field having an intensity of 20 Oe (1.6 kA/m) to 200 Oe (16.0 kA/m).

11. The iron oxide magnetic particles of claim 1, wherein the iron oxide magnetic particles are used in radiation.

Fig. 1

Fig. 2

Fe2p Scan
10 Scans, 3m20.5s, 400µm, CAE 50.0, 0.10 eV

Bi4f Scan
10 Scans, 1m5.5s, 400µm, CAE 50.0, 0.10 eV

I3d Scan
10 Scans, 2m30.5s, 400µm, CAE 50.0, 0.10 eV

## INTERNATIONAL SEARCH REPORT

| International application No. |
| --- |
| **PCT/KR2021/020042** |

| A. CLASSIFICATION OF SUBJECT MATTER |
| --- |
| **C01G 49/02**(2006.01)i; **A61K 49/18**(2006.01)i; B82Y 5/00(2011.01)n; B82Y 15/00(2011.01)n; B82Y 40/00(2011.01)n<br><br>According to International Patent Classification (IPC) or to both national classification and IPC |

| B. FIELDS SEARCHED |
| --- |
| Minimum documentation searched (classification system followed by classification symbols)<br>C01G 49/02(2006.01); A61B 19/00(2006.01); A61K 49/04(2006.01); A61K 49/06(2006.01); A61K 49/18(2006.01); B82B 3/00(2006.01); B82Y 30/00(2011.01); C01G 23/04(2006.01) |
| Documentation searched other than minimum documentation to the extent that such documents are included in the fields searched<br>Korean utility models and applications for utility models: IPC as above<br>Japanese utility models and applications for utility models: IPC as above |
| Electronic data base consulted during the international search (name of data base and, where practicable, search terms used)<br>eKOMPASS (KIPO internal) & keywords: 산화철(iron oxide), 자성 입자(magnetic particle), 6p 오비탈(6p orbital) |

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y | NAHA, P. C. et al. Dextran coated bismuth–iron oxide nanohybrid contrast agents for computed tomography and magnetic resonance imaging. Journal of Materials Chemistry B. 2014, vol. 2, no. 46, pp. 8239-8248.<br>See abstract; Synthesis and characterization of BION formulations section; figure 1; and table 2. | 1-11 |
| Y | US 2014-0194733 A1 (GOFORTH, A. et al.) 10 July 2014 (2014-07-10)<br>See paragraphs [0067], [0073], [0096] and [0144]. | 1-11 |
| Y | KR 10-2012-0091513 A (HANWHA CHEMICAL CORPORATION) 20 August 2012 (2012-08-20)<br>See abstract; and paragraph [0034]. | 4 |
| A | KR 10-2009-0000859 A (INDUSTRY-ACADEMIC COOPERATION FOUNDATION, YONSEI UNIVERSITY) 08 January 2009 (2009-01-08)<br>See entire document. | 1-11 |

| ☑ Further documents are listed in the continuation of Box C. | ☑ See patent family annex. |
| --- | --- |

| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| --- | --- |
| Date of the actual completion of the international search<br><br>**11 April 2022** | Date of mailing of the international search report<br><br>**11 April 2022** |
| Name and mailing address of the ISA/KR<br><br>**Korean Intellectual Property Office**<br>**Government Complex-Daejeon Building 4, 189 Cheongsa-ro, Seo-gu, Daejeon 35208** | Authorized officer |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2021/020042** |

**C.   DOCUMENTS CONSIDERED TO BE RELEVANT**

| Category* | Citation of document, with indication, where appropriate, of the relevant passages | Relevant to claim No. |
| --- | --- | --- |
| A | KR 10-2018-0076885 A (INDUSTRY ACADEMIC COOPERATION FOUNDATION OF YEUNGNAM UNIVERSITY) 06 July 2018 (2018-07-06)<br>See entire document. | 1-11 |

Form PCT/ISA/210 (second sheet) (July 2019)

**INTERNATIONAL SEARCH REPORT**
**Information on patent family members**

International application No.

**PCT/KR2021/020042**

| Patent document cited in search report | | | Publication date (day/month/year) | Patent family member(s) | | | Publication date (day/month/year) |
|---|---|---|---|---|---|---|---|
| US | 2014-0194733 | A1 | 10 July 2014 | CN | 103582500 | A | 12 February 2014 |
| | | | | EP | 2717934 | A1 | 16 April 2014 |
| | | | | EP | 3492116 | A1 | 05 June 2019 |
| | | | | US | 10071174 | B2 | 11 September 2018 |
| | | | | WO | 2012-170569 | A1 | 13 December 2012 |
| KR | 10-2012-0091513 | A | 20 August 2012 | CN | 103347543 | A | 09 October 2013 |
| | | | | CN | 103347543 | B | 16 November 2016 |
| | | | | EP | 2673006 | A2 | 18 December 2013 |
| | | | | EP | 2673006 | B1 | 30 December 2020 |
| | | | | JP | 2014-511324 | A | 15 May 2014 |
| | | | | JP | 5701408 | B2 | 15 April 2015 |
| | | | | KR | 10-1642903 | B1 | 27 July 2016 |
| | | | | US | 2013-0323182 | A1 | 05 December 2013 |
| | | | | US | 9352058 | B2 | 31 May 2016 |
| | | | | WO | 2012-108648 | A2 | 16 August 2012 |
| | | | | WO | 2012-108648 | A3 | 01 November 2012 |
| KR | 10-2009-0000859 | A | 08 January 2009 | KR | 10-0924786 | B1 | 03 November 2009 |
| KR | 10-2018-0076885 | A | 06 July 2018 | KR | 10-1882589 | B1 | 26 July 2018 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WUST et al.** *Lancet Oncology,* 2002, vol. 3, 487-497